# EUROPÄISCHE PATENTSCHRIFT

(11) **EP 3 839 958 B1**
(45) Veröffentlichungstag und Bekanntmachung des Hinweises auf die Patenterteilung: **25.10.2023**
(21) Anmeldenummer: 19218829.0
(22) Anmeldetag: 20.12.2019
(51) Int. Cl.: G16B 25/20, G16B 40/10, G16H 50/00, C12Q 1/6851, C12Q 1/686

(54) **VERFAHREN UND VORRICHTUNG ZUR QUALITATIVEN AUSWERTUNG VON REAL-TIME PCR DATEN**
METHOD AND DEVICE FOR QUALITATIVE EVALUATION OF REAL-TIME PCR DATA
PROCÉDÉ ET DISPOSITIF D'ÉVALUATION QUALITATIVE DE DONNÉES PCR EN TEMPS RÉEL

(43) Veröffentlichungstag der Anmeldung: 23.06.2021
(73) Patentinhaber: Euroimmun Medizinische Labordiagnostika AG, 23560 Lübeck (DE)
(72) Erfinder: CHRISTODOULOS, Filippis, 23847 Kastorf (DE); STELLER, Ulf, 21244 Buchholz (DE); SCHILLING, Fiona, 23556 Lübeck (DE)

(56) Entgegenhaltungen:
- WO-A1-2019/168261
- WO-A2-2006/108205
- US-A1- 2006 008 809

## Beschreibung

Real-Time PCR (Polymerase Chain Reaction), auch Echtzeit Polymerase-Kettenreaktion genannt, ist ein Verfahren zum Nachweis spezifischer Nukleinsäuren, z.B. DNA (Desoxyribonukleinsäure) oder RNA (Ribonukleinsäure), in einer Probe. Eine solche Probe ist vorzugsweise eine biologische Probe.

Im Zuge einer PCR werden relevante, charakteristische Teilstücke von Nukleinsäuren vervielfältigt. Hierbei legen zwei sogenannte Primer, auch Starter-DNA Moleküle genannt, fest, welcher Bereich einer Nukleinsäure kopiert werden soll. Enthält die Probe einen zu den Primern passenden Nukleinsäure-Abschnitt, welcher auch Zielsequenz genannt wird, so können die Primer daran binden und es kann im Zuge eines Zyklus eine kopierte Zielsequenz hergestellt werden. Der PCR Prozess durchläuft mehrere Zyklen, wobei im Zuge eines Zyklus unterschiedliche Temperaturbedingungen in der Probe herrschen und unterschiedliche Reaktionen ablaufen. Durch die Prozessierung mehrerer Zyklen erfolgt dann bei Vorliegen von zu der Nukleinsäure passenden Primern eine Vervielfältigung der Zielsequenz.

Im Falle eines spezifischen Nachweises von RNA in einer Probe wird zunächst die RNA mittels des Enzyms reverse Transkriptase in DNA umgeschrieben. Dieser Schritt entfällt bei einem spezifischen Nachweis von DNA. Innerhalb eines Zyklus wird dann in einem ersten Schritt, der sogenannten Denaturierung, die Probe auf ungefähr 94 bis 98 °C erhitzt, um die DNA-Stränge voneinander zu trennen. In einem zweiten Schritt, der Primerhybridisierung, wird dann für einen bestimmten Zeitraum die Temperatur auf einem vorbestimmten Wert gehalten, welcher beispielsweise 55-65 °C beträgt. In diesem zweiten Schritt binden dann die Primer an die passenden DNA Abschnitte. In einem dritten Schritt, der Elongation, füllt die DNA-Polymerase die fehlenden Stränge mit freien Nukleotiden auf. Für einen vorbestimmten Zeitraum von beispielsweise 30 Sekunden herrschen dann Temperaturbedingungen von ungefähr 68-72 °C in der Probe. Gegebenenfalls können Primerhybridisierung und Elongation auch ein zusammengefasster Schritt innerhalb eines Zyklus sein mit identischen Reaktionsbedingungen.

Im Rahmen einer Real-Time PCR werden neben spezifischen Primern entweder DNAinterkalierende Fluoreszenzfarbstoffe (z.B. SYBR Green I) oder Sequenz-spezifische Fluoreszenz-markierte DNA-Sonden (FRET-Sonden, wie z.B. TaqMan-Sonden, Molecular Beacons, o. ä.) eingesetzt, um die DNA-Vervielfältigung in Echtzeit über die Fluoreszenzzunahme zu verfolgen. Am Ende eines jeden PCR Zyklus wird die Fluoreszenzintensität optisch gemessen. Eine Zunahme der Fluoreszenzintensität erfolgt nur dann, wenn eine Vervielfältigung der DNA erfolgt ist. Wenn die bestimmten Nukleinsäure-Zielsequenzen in der Probe fehlen, können die Primer und die fluoreszenzmarkierten DNA-Sonden oder Fluoreszenzfarbstoffe nicht binden: es findet keine DNA-Vervielfältigung statt und somit auch keine Erhöhung des Fluoreszenzsignals bzw. der Fluoreszenzintensität.

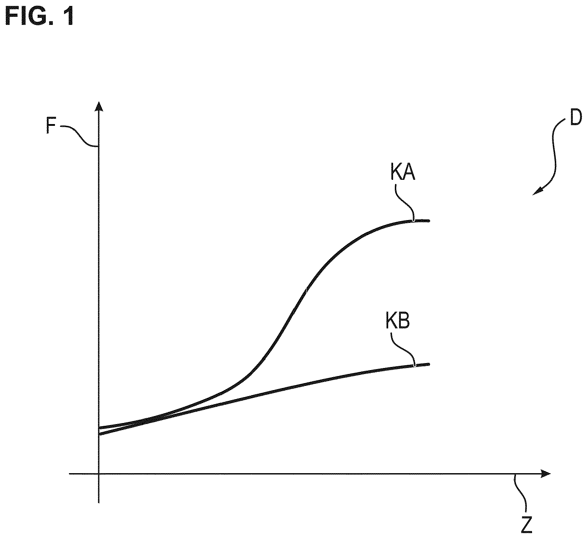

Die Figur 1 zeigt hierzu beispielhaft ein Diagramm D, in welchem eine Fluoreszenzintensität F über einer Zykluszeit Z aufgetragen ist. Die erste Amplifikationskurve KA repräsentiert das Fluoreszenzsignal F in dem Fall, dass eine zu den spezifischen Primern und ggf. fluoreszenzmarkierten DNA-Sonden passende Zielsequenz, vorzugsweise ein passender DNA Abschnitt, in dem Probenmaterial vorhanden ist. Die Fluoreszenzintensität F der Kurve KA steigt daher in sigmoidaler Weise an.

Die Figur 1 zeigt hierzu ferner eine zweite Amplifikationskurve KB, welche den Fall repräsentiert, dass in der Probe kein passender Nukleinsäure-Abschnitt als Zielsequenz passend zu den spezifischen Primern und ggf. fluoreszenzmarkierten DNA-Sonden vorhanden ist. Einen leichten Anstieg der Fluoreszenz F kann man ggf. auch bei der Kurve KB aufgrund anderer Effekte beobachten.

Prinzipielle Aufgabe ist es, zu erkennen, ob eine Probe positiv ist, also ob die untersuchte Probe zu detektierendes Material bzw. eine spezifische Nukleinsäure bzw. einen spezifischen Nukleinsäureabschnitt enthält oder nicht. Es muss also eine Amplifikationskurve als eine Positivkurve oder eine Negativkurve klassifiziert werden. Eine Positivkurve weist einen näherungsweise sigmoidalen Kurvenverlauf auf. Eine Positivkurve weist insbesondere eine Initiationsphase auf, ferner eine lineare Phase sowie eine Plateauphase. Eine Negativkurve weist einen näherungsweise linearen Kurvenverlauf auf.

Die Entscheidung, eine Amplifikationskurve als eine Positivkurve zu klassifizieren, ist im Sinne dieser Anmeldung gleichbedeutend mit der Entscheidung, dass festgestellt wird, dass das untersuchte Probenmaterial eine spezifische Nukleinsäure bzw. einen spezifischen Nukleinsäureabschnitt enthält. Die spezifische Nukleinsäure kann auch als eine zu detektierende Nukleinsäure bzw. als ein zu detektierender Nukleinsäureabschnitt bezeichnet werden. Die Entscheidung, eine Amplifikationskurve als eine Negativkurve zu klassifizieren, ist im Sinne dieser Anmeldung gleichbedeutend mit der Entscheidung, dass festgestellt wird, dass das untersuchte Probenmaterial eine spezifische Nukleinsäure bzw. einen spezifischen Nukleinsäureabschnitt nicht enthält. Die spezifische Nukleinsäure kann auch als eine zu detektierende Nukleinsäure bzw. als ein zu detektierender Nukleinsäureabschnitt bezeichnet werden.

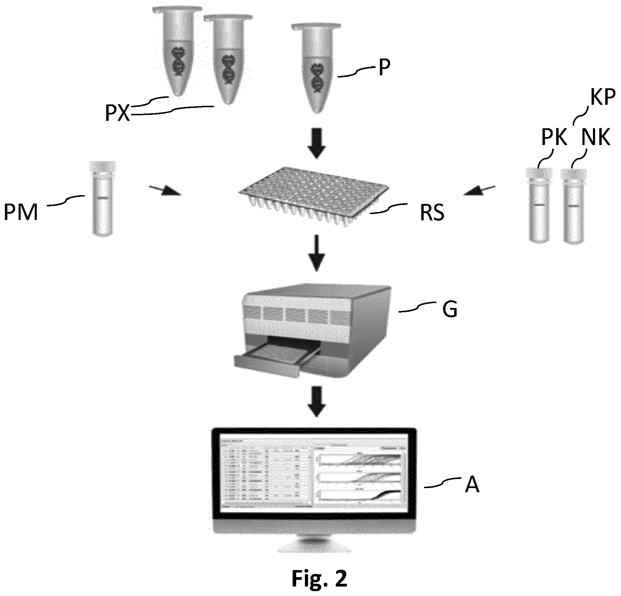

Die Figur 2 zeigt ein prinzipielles Vorgehen zum gleichzeitigen Untersuchen bzw. Prozessieren mehrerer Proben P, PX in einem gemeinsamen Testlauf bei gleichen Prozessierungsbedingungen. Im Zuge der Untersuchung einer spezifischen Probe P wird der Probe P ein PCR-Mix PM, bestehend aus spezifischen Primermaterial, Fluoreszenzfarbstoffen oder Fluoreszenz-markierten DNA-Sonden sowie weiteren Reaktionskomponenten (z.B. Puffer, Desoxyribonukleosid-Triphosphate, Salze, Polymerasen, Additive, ggf. weitere), beigemischt. Gleiches kann für weitere Proben Px durchgeführt werden. Dieser Ablauf kann als ein gemeinsamer bzw. gleichzeitiger Testlauf für mehrere unterschiedliche Proben P, PX gemeinsam bzw. gleichzeitig erfolgen. Die jeweiligen Proben P, PX befinden sich dann in jeweiligen Reaktionsgefäßen RS, welche vorzugsweise durch jeweilige Vertiefungen einer Mikrotiterplatte gegeben sind. Gleichzeitig können dann jeweilige Kontrollproben KP ebenfalls in entsprechenden jeweiligen Reaktionsgefäßen bereitgestellt werden. Eine Positivkontrolle PK bzw. Positivkontrollprobe weist dann sicher das zu detektierende Material bzw. den zu detektieren Nukleinsäure-Abschnitt (Zielsequenz) auf. Auch der Positivkontrollprobe PK werden PCR-Mix PM beigemischt. Gleiches wird für eine Negativkontrollprobe NK durchgeführt, welche sicher nicht den zu detektierenden Nukleinsäure-Abschnitt (Zielsequenz) bzw. nicht das zu detektierende Material aufweist. Auch die Negativkontrollprobe wird dann in ein entsprechendes Reaktionsgefäß überführt.

Solchermaßen befüllte Reaktionsgefäße RS bzw. die Mikrotiterplatte können/kann dann in eine temperierbare Halterung des Real-Time PCR-Gerätes G eingelegt werden. In ebengenanntem Real-Time PCR Gerät G wird dann ein Temperaturverlauf der Polymerase-Kettenreaktion in den zuvor genannten unterschiedlichen Schritten für mehrere Zyklen zur Prozessierung der Proben P, NK, PK unter gleichen Prozessierungsbedingungen durchlaufen. Es wird dann nach jedem Zyklus für jede Probe P, NK, PK ein entsprechender Fluoreszenzwert gemessen und aufgenommen. Die entsprechenden sich ergebenden Real-Time PCR-Amplifikationskurven der jeweiligen Proben P, NK, PK können dann in einer Anzeige A angezeigt werden. Die Gesamtheit aus der Probe P, der Negativkontrollprobe NK und der Positivkontrollprobe PK kann also in einem gemeinsamen Testlauf unter gemeinsamen Bedingungen prozessiert werden.

Wie zuvor erwähnt, ist es die Aufgabe, eine Real-Time PCR-Amplifikationskurve der Probe P als eine Negativkurve oder eine Positivkurve zu klassifizieren. Alternativ gesprochen kann dies ausgedrückt werden als die Entscheidung, ob die Probe P bestimmtes zu detektierendes Material bzw. einen bestimmten zu detektierenden Nukleinsäure-Abschnitt, auch Nukleinsäure-Zielsequenz genannt, enthält oder nicht.

Hierzu offenbart US 2006/008809 A1 ein Verfahren, bei dem für gefensterte Abschnitte einer Amplifikationskurve jeweils eine "Local Quality Value" (LQV)-Metrik bestimmt wird, in die sowohl die Steigung als auch das Gütemaß der linearen Regression (R-Wert) eingehen. Eine finale Klassifizierung als Negativkurve oder Positivkurve erfolgt anhand einer "Quality Score" (QS)-Metrik, welches jener LQV-Wert ist, der das Maximum aller LQV-Werte ergibt.
Demgegenüber wird hier das folgende erfindungsgemäße Verfahren vorgeschlagen.

Die erfindungsgemäße Aufgabe wird gelöst durch ein vorgeschlagenes computerimplementiertes Verfahren zur qualitativen Auswertung von Real-Time PCR Daten, wobei eine Klassifikation einer Real-Time PCR-Amplifikationskurve einer zugehörigen Probe als eine Negativkurve oder als eine Positivkurve erfolgt. Das Verfahren weist unterschiedliche Schritte auf. In einem Schritt wird eine zu klassifizierende Real-Time PCR-Amplifikationskurve einer zugehörigen Probe bereitgestellt. Dieses erfolgt vorzugsweise in Form von digitalen Daten. In einem nächsten Schritt erfolgt ein Bestimmen eines Gütemaßes, welches eine Ähnlichkeit der zu klassifizierenden Real-Time PCR-Amplifikationskurve zu einer Geraden indiziert, als ein Gütemaß einer linearen Regression in Bezug auf die zu klassifizierenden Real-Time PCR-Amplifikationskurve, In einem weiteren Schritt erfolgt ein Bestimmen eines ersten Kriteriums durch Überprüfen, ob das Gütemaß der linearen Regression den ersten Vorgabewert unterschreitet. In einem weiteren Schritt erfolgt ein Bestimmen einer Wertefolge, welche eine Steigung der zu klassifizierenden Real-Time PCR-Amplifikationskurve indiziert, sowie ein Bestimmen eines zweiten Kriteriums, ob die Wertefolge einen zweiten Vorgabewert übersteigt. Schließlich erfolgt eine Klassifikation der Real-Time PCR-Amplifikationskurve der zugehörigen Probe als eine Positivkurve in dem Fall, dass alle Kriterien erfüllt sind.

Das erfindungsgemäße Verfahren birgt den Vorteil, dass nicht nur darauf abgestellt wird, ob als erstes Kriterium eine Ähnlichkeit der Amplifikationskurve gegenüber einer Geraden gegeben ist, bzw. ob ein Gütemaß, welches indiziert, dass die Kurve linear verläuft, mit einem Vorgabewert verglichen wird. Es erfolgt eben erfindungsgemäß auch noch das Bestimmen der Wertefolge, welche die Steigung der Amplifikationskurve indiziert sowie die Überprüfung eines zweiten Kriteriums, ob diese Steigung einen Vorgabewert übersteigt. Hierdurch wird also als zusätzliches Kriterium betrachtet, ob die Steigung der Amplifikationskurve hinreichend stark ist in einem der betrachteten Zyklusbereiche. Mit anderen Worten: Es wird also nicht nur überprüft, ob die Amplifikationskurve von einer Geraden hinreichend abweicht sondern auch, ob die Steigung der Amplifikationskurve hinreichend groß ist. Hierdurch werden zwei Kriterien verknüpft, um zu überprüfen, ob die Amplifikationskurve an einer einfachen Geraden abweicht.

Hierdurch wird eine zuverlässigere Klassifikation der Amplifikationskurve als eine Positivkurve erreicht als im Stand der Technik, gemäß welchem lediglich ein Vergleich der Amplifikationskurve mit einer Geraden betrachtet wird.

Vorteilhafte Ausführungsformen der Erfindung sind Gegenstand der abhängigen Ansprüche und werden in der folgenden Beschreibung unter teilweiser Bezugnahme auf die Figuren näher erläutert.

Das Verfahren weist vorzugsweise ein Bestimmen eines weiteren Kriteriums auf, ob die Wertefolge, welche eine Steigung der zu klassifizierenden Real-Time PCR-Amplifikationskurve indiziert, einen dritten Vorgabewert übersteigt. Ferner weist das Verfahren vorzugsweise ein Klassifizieren der Real-Time PCR-Amplifikationskurve der zugehörigen Probe als eine Positivkurve in dem Fall, dass alle Kriterien erfüllt sind.

Durch Bestimmen dieses weiteren Kriteriums auf Basis des dritten Vorgabewertes wird in vorteilhafter Weise überprüft, ob die Steigung weitere Kriterien erfüllt. Dieses erlaubt eine noch sicherere Bestimmung des Ergebnisses bzw. sicherere Klassifikation der Amplifikationskurve der Probe als eine Positivkurve.

Ferner weist das Verfahren vorzugsweise auf
- ein Bereitstellen einer Real-Time PCR-Amplifikationskurve einer Positivkontrollprobe sowie einer Real-Time PCR-Amplifikationskurve einer Negativkontrollprobe,
- ein Bestimmen eines ersten Nebenkriteriums, ob der letzte Fluoreszenzwert der Real-Time PCR-Amplifikationskurve der Positivkontrollprobe einen Minimalwert überschritten hat,
- ein Bestimmen eines zweiten Nebenkriteriums, ob der letzte Fluoreszenzwert der Real-Time PCR-Amplifikationskurve der Negativkontrollprobe einen Maximalwert nicht überschritten hat,
- sowie ein Klassifizieren der Real-Time PCR-Amplifikationskurve der zugehörigen Kurve als nicht valide, falls wenigstens eines der Nebenkriterien nicht erfüllt wird.

Prinzipiell ist es möglich, dass während der Durchführung der Real-Time PCR Prozessierungsfehler, zum Beispiel hinsichtlich des Temperaturverlaufes, erfolgen können, welche sich zum Beispiel dadurch bemerkbar machen würden, dass der letzte Fluoreszenzwert der Amplifikationskurve der Positivkontrollprobe einen Minimalwert nicht überschreitet. Ebenso darf eigentlich der letzte Fluoreszenzwert der Amplifikationskurve der Negativkontrollprobe einen bestimmten Maximalwert nicht überschreiten, welches als Bedingung verletzt werden könnte, falls die Negativprobe mit der nachzuweisenden Zielsequenz kontaminiert wäre. Dann würde nämlich der letzte Fluoreszenzwert der Amplifikationskurve der Negativkontrollprobe Vorgabewert bzw. den Maximalwert übersteigen.

Daher kann durch diese weiteren Kriterien eine Fehlprozessierung im Zuge der PCR detektiert werden und dann vermieden werden, die Amplifikationskurve der zugehörigen Probe als valide zu klassifizieren. Mit anderen Worten: Die bevorzugte Ausgestaltung des Verfahrens ist eben vorteilhaft, da hierbei überprüft werden kann, ob die Probe der Positivkontrollprobe und eben auch die zugehörige Probe der zu klassifizierenden Amplifikationskurve im Zuge der Real-Time PCR ordnungsgemäß prozessiert wurden. Wurde ein ordnungsgemäßes Prozessieren der Positivkontrollprobe und der Negativkontrollprobe nicht festgestellt, weil wenigstens eines der Nebenkriterien nicht erfüllt wird, so kann dann darauf geschlossen werden, dass die Real-Time PCR-Amplifikationskurven der zugehörigen Proben als nicht valide zu klassifizieren sind.

Erfindungsgemäß wird das Gütemaß als ein Gütemaß einer linearen Regression in Bezug auf die zu klassifizierenden Real-Time PCR-Amplifikationskurve bestimmt. Ferner wird erfindungsgemäß das erste Kriterium bestimmt durch Überprüfen, ob das Gütemaß der linearen Regression den ersten Vorgabewert unterschreitet.

Ferner weist das Verfahren vorzugsweise auf
- ein Bestimmen des letzten Fluoreszenzwertes der zu klassifizierenden Real-Time PCR-Amplifikationskurve
- sowie ein Klassifizieren der Real-Time PCR-Amplifikationskurve der zugehörigen Probe als eine Positivkurve in Abhängigkeit des letzten Fluoreszenzwertes der zu klassifizierenden Real-Time PCR-Amplifikationskurve.

Durch das Klassifizieren der Amplifikationskurve der zugehörigen Probe als eine Positivkurve in Abhängigkeit des letzten Fluoreszenzwertes kann abermals eine Qualität des Klassifikationsergebnisses erhöht werden.

Vorgeschlagen wird ferner eine Vorrichtung zur qualitativen Auswertung von Real-Time PCR Daten. Die Vorrichtung zur qualitativen Auswertung von Real-Time PCR Daten weist eine Speichereinheit zum Bereitstellen einer zu klassifizierenden Real-Time PCR-Amplifikationskurve einer zugehörigen Probe auf. Ferner weist die Vorrichtung eine Recheneinheit auf zum Bestimmen eines Gütemaßes, welches eine Ähnlichkeit der zu klassifizierenden Real-Time PCR-Amplifikationskurve zu einer Geraden indiziert, als ein Gütemaß einer linearen Regression in Bezug auf die zu klassifizierenden Real-Time PCR-Amplifikationskurve. Die Recheneinheit ist ferner ausgebildet zum Bestimmen eines ersten Kriteriums durch Überprüfen, ob das Gütemaß der linearen Regression den ersten Vorgabewert unterschreitet.

Die Recheneinheit ist ferner ausgebildet zum Bestimmen einer Wertefolge, welche eine Steigung der zu klassifizierenden Real-Time PCR-Amplifikationskurve indiziert, sowie ferner zum Bestimmen eines zweiten Kriteriums, ob die Wertefolge einen ersten Vorgabewert übersteigt. Die Recheneinheit nimmt eine Klassifikation der Real-Time PCR-Amplifikationskurve der zugehörigen Probe als eine Negativkurve oder als eine Positivkurve vor, wobei die Recheneinheit die Real-Time PCR-Amplifikationskurve der zugehörigen Probe in dem Fall als eine Positivkurve klassifiziert, dass alle Kriterien erfüllt sind. Die Vorrichtung ist vorzugsweise in weiteren Formen ausgestaltet, welche zu den weiteren vorzugsweisen Ausgestaltungen des vorgeschlagenen Verfahrens korrespondieren.

Vorgeschlagen wird ferner ein Computerprogrammprodukt, umfassend Befehle, die bei der Ausführung des Programms durch einen Computer diesen veranlassen, das computerimplementierte Verfahren auszuführen.

Im Folgenden wird die Erfindung anhand spezieller Ausführungsformen ohne Beschränkung des allgemeinen Erfindungsgedankens anhand von Figuren näher erläutert.

Dabei zeigen:
Figur 1 beispielhafte Amplifikationskurven,
Figur 2 das Prinzip der Verwendung mehrerer Proben sowie von Kontrollproben und Reagenzien in einem Testlauf,
Figur 3 bevorzugte Schritte zur Durchführung einer bevorzugten Ausführungsform des vorgeschlagenen Verfahrens,
Figur 4 weitere bevorzugte Schritte zur Durchführung einer bevorzugten Ausführungsform des vorgeschlagenen Verfahrens,
Figur 5 beispielhafte Amplifikationskurven einer Positivkontrollprobe und einer Negativkontrollprobe,
Figur 6 Amplifikationskurven von Proben,
Figur 7 modifizierte Amplifikationskurven sowie eine Gerade,
Figur 8 Kurven, welche ein Steigungsverhalten der Amplifikationskurven aus Figur 6 repräsentieren,
Figur 9 Wertefolgen, welche jeweilige Steigungen der jeweiligen Amplifikationskurven aus der Figur 6 indizieren,
Figur 10 die Amplifikationskurven aus Figur 6 sowie einen Vorgabewert,
Figur 11 eine bevorzugte Ausführungsform der vorgeschlagenen Vorrichtung,
Figur 12 unterschiedliche Tabellen unterschiedlicher Testergebnisse für unterschiedliche Testsätze.

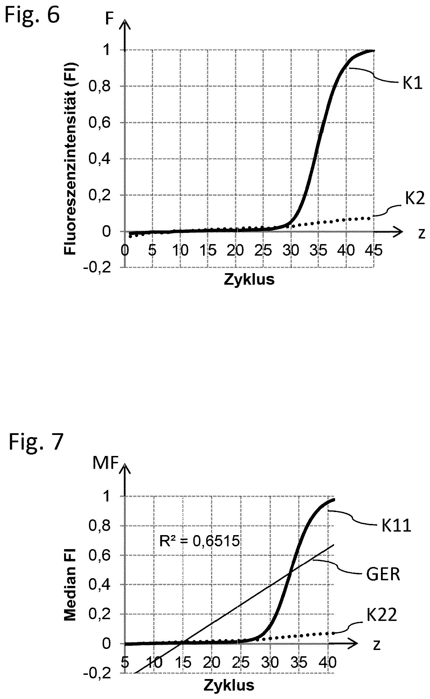

Die Figur 6 zeigt unterschiedliche Amplifikationskurven K1, K2. Aufgetragen ist die Fluoreszenzintensität F über der Zykluszeit Z. Die Kurve K1, welche eine sigmoidale Steigung aufweist, ist als Positivkurve zu klassifizieren. Die Kurve K2, welche keinen oder eher einen frühen linearen Anstieg aufweist, ist als negativ zu klassifizieren. Die Kurven K1, K2 sind auf einen Wertebereich 0...1 normiert.

Es wird im Folgenden eine Verarbeitung bzw. Auswertung der Kurve K1 als Beispiel im Detail genau näher erläutert. Entsprechende Verarbeitungswerte für die Kurve K2 sind ebenfalls in den weiteren Figuren illustriert.

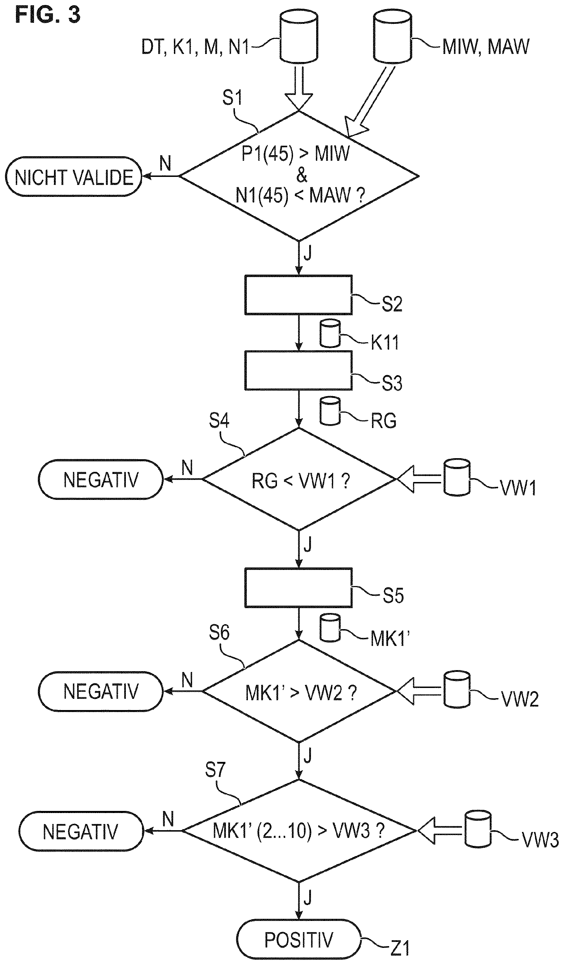

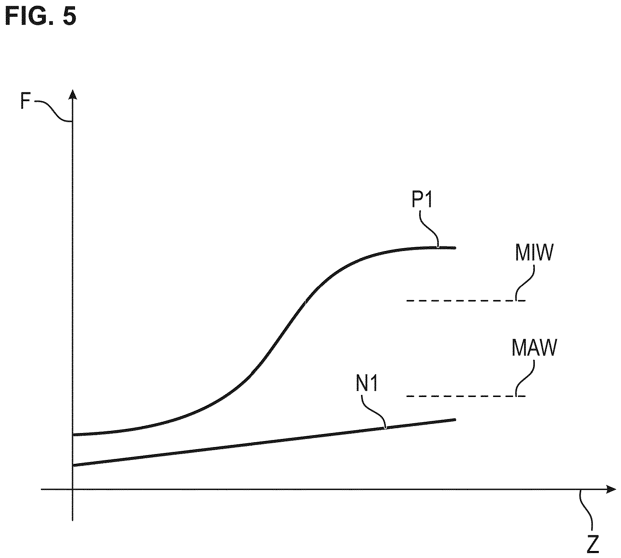

Gemäß der Figur 3 kann die Amplifikationskurve K1 als ein Datensatz DT bereitgestellt werden. Die Figur 5 zeigt eine beispielhafte Illustration einer Amplifikationskurve P1 einer Positivkontrollprobe aus dem gleichen Testlauf sowie eine beispielhafte Illustration einer Amplifikationskurve N1 einer Negativkontrollprobe aus dem gleichen Testlauf. Vorzugsweise werden ferner die Amplifikationskurve P1 der Positivkontrollprobe und die Amplifikationskurve N1 der Negativkontrollprobe aus dem gleichen Testlauf bereitgestellt, vorzugsweise mittels des gleichen Datensatzes DT oder eines weiteren hier nicht genauer dargestellten Datensatzes. Vorzugsweise können bereitgestellte Amplifikationskurven der Positivkontrollprobe PK und der Negativkontrollprobe NK auch aus einem anderen Testlauf als die Amplifikationskurven der einen Probe P oder auch der mehreren Proben P stammen, sofern diese unter gleichen Prozessierungsbedingungen erzeugt wurden, und mittels eines weiteren hier nicht genauer dargestellten Datensatzes bereitgestellt werden.

Vorzugsweise werden ebenfalls Vorgabewerte MIW, MAW bereitgestellt.

In einem ersten Schritt S1 können dann vorzugsweise die Amplifikationskurven P1, N1 aus der Figur 5 anhand der Vorgabewerte MIW sowie MAW überprüft werden. Hierbei wird überprüft, ob die Amplifikationskurve P1 der Positivkontrollprobe an dem letzten Zyklusindex bzw. mit ihrem letzten Fluoreszenzwert eine minimale Endfluoreszenz, gegeben durch den des Vorgabewert MIW, überschreitet. Es wird hierdurch z.B. überprüft, ob die Prozessierungsbedingungen innerhalb des Gerätes G aus der Figur 2 entsprechend eingehalten wurden. Es kann hierdurch auch überprüft werden, ob z.B. die PCR-Reagenzien noch funktioniert haben und ob diese richtig zusammenpipettiert wurden. Ebenso kann überprüft werden, ob die Ansätze richtig pipettiert wurden.

Ferner wird auch überprüft, ob die Amplifikationskurve N1 der Negativkontrollprobe an dem letzten Zyklusindex bzw. mit ihrem letzten Fluoreszenzwert eine maximale Endfluoreszenz, gegeben durch den Vorgabewert MAW, nicht übersteigt. Hierdurch können Reagenzienkontaminationen z.B. der PCR-Reagenzien oder der zur Probenextraktion verwendeten Reagenzien, mit der nachzuweisenden Zielsequenz aufgedeckt werden. Wäre der Ansatz der Negativkontrollprobe kontaminiert, so würde der letzte Fluoreszenzwert der Kurve N1 den Vorgabewert MAW übersteigen.

Es wird also ein erstes Nebenkriterium, ob der letzte Fluoreszenzwert der Amplifikationskurve der Positivkontrollprobe einen Minimalwert überschritten hat, bestimmt sowie ferner ein zweites Nebenkriterium, ob der letzte Fluoreszenzwert der Amplifikationskurve der Negativkontrollprobe einen Maximalwert nicht überschritten hat. Die Kurve P1 der Positivkontrollprobe und die Kurve K1 der Negativkontrollprobe werden dann als valide angesehen und dann eben beide Nebenkriterien als erfüllt angesehen. Sind beide Nebenkriterien erfüllt, so wird darauf geschlossen, dass auch die Amplifikationskurve K1 der zugehörigen Probe P prinzipiell valide ist und dann somit zum Schritt S2 übergegangen. Ist wenigstens eines der Nebenkriterien nicht erfüllt, so wird darauf geschlossen, dass die Amplifikationskurve K1 der zugehörigen Probe nicht valide ist und dann eben das Verfahren abgebrochen.

Weiterer Nebenkriterien können vorzugsweise für weitere vorzugsweise Kontrollproben (z.B. eine interne Kontrolle oder eine endogene Kontrolle) und zugehörige Kurven bestimmt und geprüft werden. Eine interne Kontrolle kann bspw. notwendig sein um aufzeigen, dass eine Extraktion von Nukleinsäuren aus einer Patientenprobe erfolgreich verlaufen ist. Es erfolgt aber nur vorzugsweise eine Ausführung dieser weiteren Nebenkriterien in dem weiteren Verfahren.

Die Figur 6 zeigt die in Betracht gezogene Amplifikationskurve K1 der zugehörigen Probe. Ferner zeigt die Figur 6 eine weitere Amplifikationskurve K2 als Beispiel einer Negativkurve.

In einem Schritt S2 erfolgt vorzugsweise eine Medianfilterung der Kurve K1 aus der Figur 6, sodass sich die modifizierte Amplifikationskurve K11 aus der Figur 7 ergibt. Analoges gilt für die Kurven K2 bzw. K21 aus den Figuren 6 bzw. 7.

Die Medianfilterung erfolgt vorzugsweise derart, dass immer drei Werte der Amplifikationskurve K1 einem Medianfilter zugeführt werden und der sich ergebende Wert dann einen Wert der Kurve K11 definiert. Das Medianfilter kann als gleitendes Filter implementiert werden.

Es wird dann in einem Schritt S3 ein Gütemaß RG bestimmt, welches eine Ähnlichkeit der modifizierten Real-Time PCR-Amplifikationskurve K11 zu einer Geraden indiziert. Vorzugsweise wird in dem Schritt S3 das Gütemaß RG als ein Gütemaß einer linearen Regression in Bezug auf die zu klassifizierenden Real-Time PCR-Amplifikationskurve bestimmt. Erfindungsgemäß erfolgt dies durch Bestimmen einer linearen Regressionsgeraden GER, gezeigt in Figur 7, sowie durch Bestimmen des Gütemaßes RG als ein Bestimmtheitsmaß R², auch Determinationskoeffizient genannt, welches insbesondere ein Maß zur Beurteilung der Anpassungsgüte zu klassifizierenden Real-Time PCR-Amplifikationskurve an die lineare Regressionsgerade GER ist. Eine entsprechende Regressionsgerade ist in der Figur 7 nicht für die Kurve K21 eingetragen.

Das Gütemaß RG kann als ein Gütemaß einer Ähnlichkeit der modifizierten Real-Time PCR-Amplifikationskurve K11 zu einer Geraden und eben auch als ein Gütemaß einer Ähnlichkeit der zu klassifizierenden Real-Time PCR-Amplifikationskurve K1 zu einer Geraden aufgefasst werden.

In einem Schritt S4 wird dann ein erster Vorgabewert VW1 bereitgestellt. Es wird dann ein erstes Kriteriums mittels eines Vergleichs des Gütemaßes RG mit einem ersten Vorgabewert VW1 bestimmt. Es wird erfindungsgemäß als das erste Kriterium bestimmt, ob das Gütemaß RG den Vorgabewert VW1 unterschreitet. Besonders bevorzugt wird als das erste Kriterium bestimmt, ob das Bestimmtheitsmaß R2 der linearen Regression in Bezug auf die zu klassifizierende Amplifikationskurve K1 den Vorgabewert VW1 unterschreitet. Ist das erste Kriterium nicht erfüllt, so wird die Amplifikationskurve K1 als negativ klassifiziert. Der erste Vorgabewert VW1 ist vorzugsweise ein Wert zwischen 0,92 und 0,99.

Ist das erste Kriterium erfüllt, so wird dann weiter zu dem Schritt S5 verfahren. Die Kurve K1 wird also als potentiell positiv angesehen.

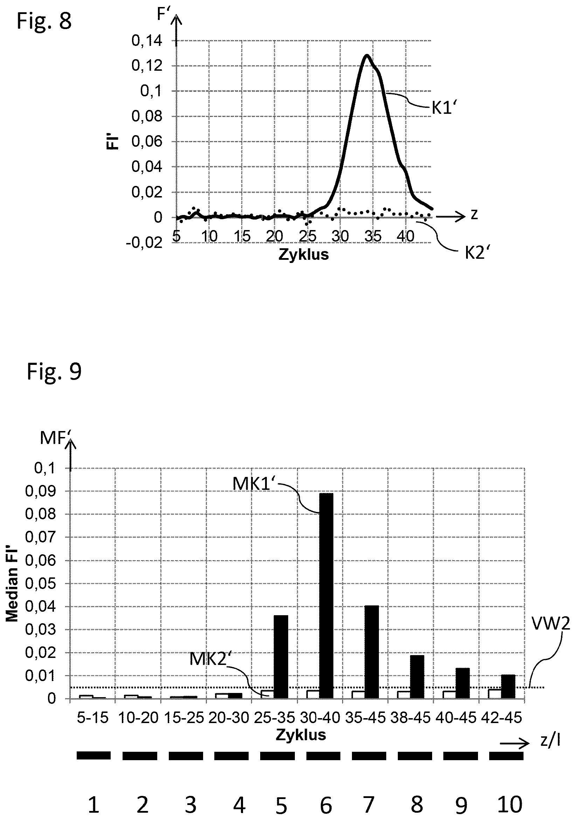

In dem Schritt S5 wird zunächst eine Steigungskurve K1', siehe Figur 8, zu der Kurve K1 aus der Figur 6 bestimmt. Beispielhaft ist in Figur 8 auch noch die Steigungskurve K2' für die Kurve K2 aus der Figur 6 indiziert.

Ferner wird die Steigungskurve K1' aus der Figur 8 in dem Schritt S5 einer Medianfilterung unterzogen, sodass sich gefilterte Steigungswerte MF' ergeben. Das Balkendiagramm aus Figur 9 zeigt hierzu eine sich ergebende Wertefolge MK1', welche eine Steigung K' der zu klassifizierenden Amplifikationskurve K1 aus der Figur 6 indiziert. Es ist ferner auch eine Wertefolge MK2' illustriert, welche eine Steigung K2' der Kurve K2 aus der Figur 6 indiziert. Die Figur 9 zeigt hierbei für Indexwerte I von 1 bis 10 und darüber jeweils entsprechend dargestellte die entsprechenden Zykluswerte, welche mittels einer Medianfilterung zusammengefasst werden. Ferner ist der zweite Vorgabewert VW2 eingezeichnet.

Es wird dann in einem Schritt S6 ein zweiter Vorgabewert VW2 bereitgestellt. Der zweite Vorgabewert ist vorzugsweise ein Wert zwischen 0,002 und 0,01.

In einem Schritt S6 wird dann als ein zweites Kriterium bestimmt, ob die Wertefolge MK1' den zweiten Vorgabewert VW2 übersteigt. Ist dies nicht der Fall, so wird die Kurve K1 als eine Negativkurve klassifiziert. Ist das zweite Kriterium erfüllt, dann wird hin zu einem Schritt S7 übergegangen, sodass die Kurve K1 als eine potentielle Positivkurve klassifiziert wird.

Es lässt sich also zusammenfassend feststellen, dass in dem erfindungsgemäßen Verfahren eine Amplifikationskurve K1 bereitgestellt wird, dass dann ein Gütemaß bestimmt wird, welches eine Ähnlichkeit der zu klassifizierenden Real-Time PCR-Amplifikationskurve zu einer Geraden indiziert, dass dann ein erstes Kriterium mittels eines Vergleichs des Gütemaßes mit einem ersten Vorgabewert bestimmt wird, dass ferner eine Wertefolge MK1' bestimmt wird, welche eine Steigung der zu klassifizierenden Amplifikationskurve K1 indiziert, und dass ferner ein zweites Kriterium bestimmt wird, ob die Wertefolge MK1' einen zweiten Vorgabewert VW2 übersteigt.

Erfindungsgemäß wird das Gütemaß als ein Gütemaß einer linearen Regression in Bezug auf die zu klassifizierenden Real-Time PCR-Amplifikationskurve bestimmt. Ferner wird erfindungsgemäß das erste Kriteriums durch eine Überprüfung bestimmt, ob das Gütemaß der linearen Regression den ersten Vorgabewert unterschreitet, das ein erstes Kriterium, ob ein Gütemaß einer linearen Regression in Bezug auf die zu klassifizierende Amplifikationskurve K1 einen ersten Vorgabewert VW1 unterschreitet.

Ferner kann zusammenfassend festgestellt werden, dass in dem erfindungsgemäßen Verfahren die Amplifikationskurve K1 dann als eine Positivkurve klassifiziert wird, falls das erste und das zweite Kriterium erfüllt sind.

Die Wertefolge MK2' in der Figur 9 indiziert entsprechende Werte bezogen auf die Kurve K2 aus der Figur 6.

In dem Schritt S7 wird dann ein dritter Vorgabewert VW3 bereitgestellt. Dieser Vorgabewert ist vorzugsweise ein Vielfaches des ersten Wertes der Wertefolge MK1'. Dieser Vorgabewert ist vorzugsweise das 1,5 bis 3-fache des ersten Wertes der Wertefolge MK1', also das 1,5 - 3-fache des Wertes MK1' (1). Es wird dann als ein Kriterium bestimmt, ob die Wertefolge MK1', welche die Steigung der zu klassifizierenden Amplifikationskurve K1 indiziert, den dritten Vorgabewert VW3 übersteigt. Hierbei wird überprüft, ob einer der Werte MK1' (2... 10) der Indizes 2 bis 10 der Folge MK1' den dritten Vorgabewert VW3 übersteigt. Ist dies der Fall, so wird hin zu dem Zustand Z1 übergegangen und die Amplifikationskurve K1 als positiv klassifiziert. Ist dies nicht der Fall, so wird entsprechend übergegangen zu dem Schritt S3 und die Amplifikationskurve K1 als negativ klassifiziert.

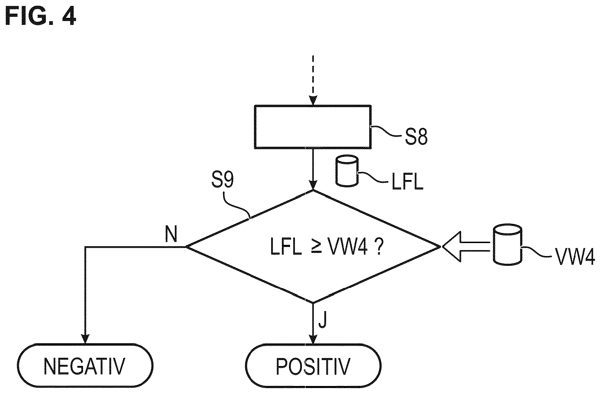

Vorzugsweise kann eine Klassifikation der Amplifikationskurve K1 der zugehörigen Probe in genauerer Weise durch Durchführung weiterer Schritte aus der Figur 4 durchgeführt werden, welche sich an den Schritt S7 aus der Figur 3 anschließen können. Eine Klassifikation der Amplifikationskurve K1 ist dann nicht nach dem Schritt S7 abgeschlossen, sondern erst nach Durchführung weiterer Schritte aus der Figur 4.

In einem Schritt S8 wird der letzte Fluoreszenzwert des letzten Zyklusindex der zu klassifizierenden Amplifikationskurve K1 bestimmt. Dieses ist in der Figur 4 der Wert LFL.

Die Klassifikation der Amplifikationskurve K1 der zugehörigen Probe erfolgt dann vorzugsweise in Abhängigkeit des letzten Fluoreszenzwertes LFL der zu klassifizierenden Amplifikationskurve K1.

Es wird dann in dem Schritt S9 der letzte Fluoreszenzwert LFL der zu klassifizierenden Kurve K1 mit einem Vorgabewert VW4 verglichen, welcher vorzugsweise einen Wert zwischen 0,05 und 0,2 beträgt. Ist der letzte Fluoreszenzwert LFL der Kurve K1 größer als der Vorgabewert VW4, so wird die Kurve K1 als positiv klassifiziert, andernfalls als negativ.

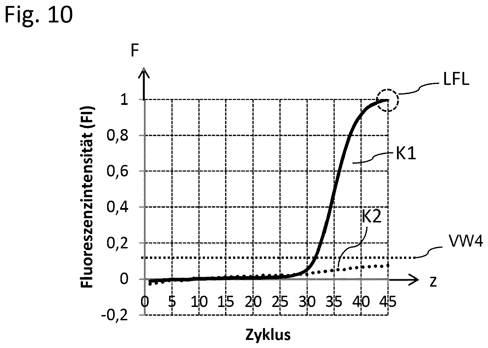

Hierzu zeigt Figur 10 die Kurven K1, K2 sowie den letzten Fluoreszenzwert LFL der Kurve K1. Ferner zeigt Figur 10 als Vergleichswert den Wert VW4, welcher vorzugsweise im Bereich von 0,05 bis 0,2 liegt. Da für die Kurve K1 dieses Kriterium erfüllt ist, wird dann in dem Schritt S9 die Kurve K1 als eine Positivkurve klassifiziert.

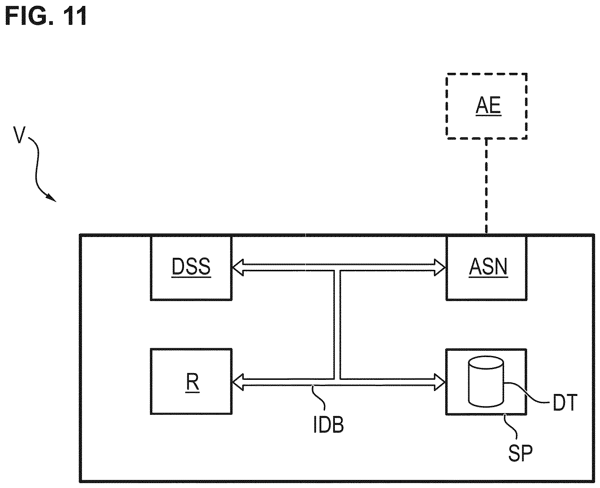

Die Figur 11 zeigt eine vorgeschlagene Vorrichtung V zur qualitativen Auswertung von Real-Time PCR Daten.

Die Vorrichtung V weist eine Recheneinheit R sowie eine Speichereinheit SP auf. Die Speichereinheit SP kann Daten DT speichern, welche vorzugsweise Daten von Amplifikationskurven repräsentieren. Vorzugsweise verfügt die Vorrichtung V über eine Datenschnittstelle DSS zum Entgegennehmen von Werten, welche Amplifikationskurven repräsentieren. Vorzugsweise ist die Datenschnittstelle DSS eine interne Schnittstelle in dem Fall, dass die Vorrichtung V selber ein PCR Gerät ist und entsprechende Amplifikationskurven bzw. deren Werte messen kann.

Vorzugsweise weist die Vorrichtung V eine Ausgabeschnittstelle ASN auf, welche vorzugsweise zu einer Ausgabeeinheit AE in Form eines Bildschirms bzw. einer Anzeige A aus der Figur 2 verbunden ist bzw. verbindbar ist.

Die Recheneinheit R und die Speichereinheit SP sind vorzugsweise über einen internen Datenbus IDB miteinander verbunden. Vorzugsweise ist die Verbindung über den internen Datenbus auch hin zu der Datenschnittstelle DSS und auch vorzugsweise zu der Ausgabeschnittstelle ASN gegeben.

Die Speichereinheit SP ist ausgebildet, die zu klassifizierende Amplifikationskurve K1 der zugehörigen Probe abzuspeichern und bereitzustellen. Ebenso ist die Speichereinheit SP vorzugsweise ausgebildet, weitere Amplifikationskurven weiterer Proben wie zum Beispiel auch der Positivkontrollprobe als auch der Negativkontrollprobe bereitzustellen.

Die Recheneinheit R ist ausgebildet zum Bestimmen eines Gütemaßes, welches eine Ähnlichkeit der zu klassifizierenden Real-Time PCR-Amplifikationskurve zu einer Geraden indiziert, sowie ferner zum Bestimmen eines ersten Kriteriums mittels eines Vergleichs des Gütemaßes mit einem ersten Vorgabewert. Erfindungsgemäß bestimmt die Recheneinheit R das Gütemaß als ein Gütemaß einer linearen Regression in Bezug auf die zu klassifizierenden Real-Time PCR-Amplifikationskurve. Vorzugsweise bestimmt die Recheneinheit R eine linearen Regressionsgeraden sowie ferner das Gütemaß als ein Bestimmtheitsmaß R², auch Determinationskoeffizient genannt, welches insbesondere ein Maß zur Beurteilung der Anpassungsgüte zu klassifizierenden Real-Time PCR-Amplifikationskurve an die lineare Regressionsgerade ist.

Die Recheneinheit bestimmt ein erstes Kriteriums mittels eines Vergleichs des Gütemaßes mit einem ersten Vorgabewert. Die Recheneinheit bestimmt erfindungsgemäß als das erste Kriterium, ob das Gütemaß den Vorgabewert unterschreitet. Besonders bevorzugt bestimmt die Recheneinheit als das erste Kriterium, ob das Bestimmtheitsmaß R² der linearen Regression in Bezug auf die zu klassifizierende Amplifikationskurve den Vorgabewert unterschreitet.

Ferner ist die Recheneinheit R ausgebildet zum Bestimmen einer Wertefolge, welche eine Steigung der zu klassifizierenden Amplifikationskurve K1 indiziert, sowie zum Bestimmen eines zweiten Kriteriums, ob die Wertefolge einen zweiten Vorgabewert übersteigt. Die Recheneinheit nimmt eine Klassifikation der Amplifikationskurve K1 der zugehörigen Probe als eine Negativkurve oder als eine Positivkurve vor. Die Recheneinheit klassifiziert die Amplifikationskurve K1 der zugehörigen Probe in dem Fall als eine Positivkurve, dass die Kriterien bezogen auf den ersten und den zweiten Vorgabewert erfüllt sind.

Vorgeschlagen wird ferner ein Computerprogrammprodukt, umfassend Befehle, die bei der Ausführung des Programms durch einen Computer diesen veranlassen, das computerimplementierte Verfahren auszuführen.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente als eine Recheneinheit kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

### ERGEBNISSE

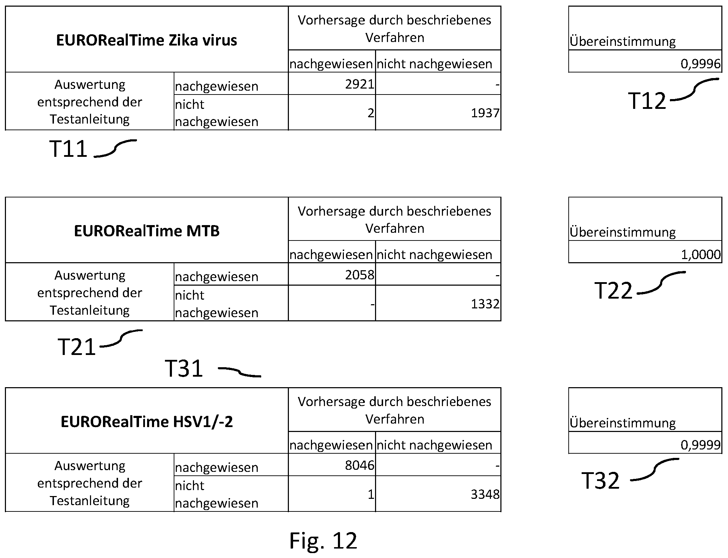

Die Figur 12 zeigt für das Produkt EURORealTime Zika virus in der Tabelle T11 experimentelle Ergebnisse durch Prozessierung gemäß der entsprechenden Testanleitung. Die entsprechenden Fluoreszenzkurven wurden mittels des beschriebenen Verfahrens ausgewertet und mit dem bisherigen manuellen Auswerteverfahren aus der entsprechenden Testanleitung verglichen. 2921 positive Probenkurven ("nachgewiesen") wurden durch das beschriebene Verfahren sicher korrekt erkannt. 1937 negative Kurven ("nicht nachgewiesen") wurden ebenfalls sicher korrekt erkannt. Bei 2 Kurven wurde die Kurvenart abweichend vom manuellen Auswerteverfahren als Positivkurve gewertet. Die Tabelle T12 zeigt hierzu die Übereinstimmung vom Grade 0,9996. Mit Übereinstimmung ist hier eine Übereinstimmung der Entscheidungen des vorgeschlagenen Verfahrens gegenüber dem tatsächlichen Vorhandensein oder Nichtvorhandensein der zu detektierenden Zielsequenz.

Entsprechende Ergebnisse für das Produkt EURORealTime MTB (Geräte: LightCycler^{®} 480 II (Roche) und 7500/7500 Fast Real-Time PCR Instrument (Applied Biosystems)) sind in den Tabellen T21 und T22 gezeigt. Hierbei ergibt sich eine Übereinstimmung vom Grade 1.

Für das Produkt EURORealTime HSV 1/-2 finden sich entsprechende Ergebnisse in den Tabellen T31 und T32. Hierbei ergibt sich eine Übereinstimmung vom Grade 0,9999. Die verwendeten Geräte waren hier: LightCycler^{®} 480 II (Roche) und 7500/7500 Fast Real-Time PCR Instrument (Applied Biosystems).

## Patentansprüche

1. Computerimplementiertes Verfahren zur qualitativen Auswertung von Real-Time PCR Daten (DT),
wobei eine Klassifikation einer Real-Time PCR-Amplifikationskurve (K1) einer zugehörigen Probe (P) als eine Negativkurve oder als eine Positivkurve erfolgt, aufweisend
- Bereitstellen der zu klassifizierenden Real-Time PCR-Amplifikationskurve (K1) der zugehörigen Probe (P),
- Bestimmen eines Gütemaßes (RG), welches eine Ähnlichkeit der zu klassifizierenden Real-Time PCR-Amplifikationskurve (K1) zu einer Geraden (GER) indiziert, als ein Gütemaß einer linearen Regression in Bezug auf die zu klassifizierenden Real-Time PCR-Amplifikationskurve (K1),
- Bestimmen eines ersten Kriteriums durch Überprüfen, ob das Gütemaß (RG) der linearen Regression den ersten Vorgabewert (VW1) unterschreitet,
- Bestimmen einer Wertefolge (MK1'), welche eine Steigung der zu klassifizierenden Real-Time PCR-Amplifikationskurve (K1) indiziert, sowie Bestimmen eines zweiten Kriteriums, ob die Wertefolge (MK1') einen zweiten Vorgabewert (VW2) übersteigt,
- Klassifizieren der Real-Time PCR-Amplifikationskurve (K1) der zugehörigen Probe (P) als eine Positivkurve in dem Fall, dass alle Kriterien erfüllt sind.

2. Computerimplementiertes Verfahren nach Anspruch 1,
ferner aufweisend
- Bestimmen eines Kriteriums, ob die Wertefolge (MK1'), welche eine Steigung der zu klassifizierenden Real-Time PCR-Amplifikationskurve indiziert, einen dritten Vorgabewert (VW3) übersteigt,
- Klassifizieren der Real-Time PCR-Amplifikationskurve der zugehörigen Probe als eine Positivkurve in dem Fall, dass alle Kriterien erfüllt sind.

3. Computerimplementiertes Verfahren nach Anspruch 1,
ferner aufweisend
- Bereitstellen einer Real-Time PCR-Amplifikationskurve (P1) einer Positivkontrollprobe (PK) sowie einer Real-Time PCR-Amplifikationskurve (N1) einer Negativkontrollprobe (NK),
- Bestimmen eines ersten Nebenkriteriums, ob der letzte Fluoreszenzwert der Real-Time PCR-Amplifikationskurve (P1) der Positivkontrollprobe (PK) einen Minimalwert (MIW) überschritten hat,
- Bestimmen eines zweiten Nebenkriteriums, ob der letzte Fluoreszenzwert der Real-Time PCR-Amplifikationskurve (N1) der Negativkontrollprobe (NK) einen Maximalwert (MAW) nicht überschritten hat,
- Klassifizieren der Real-Time PCR-Amplifikationskurve (K1) der zugehörigen Probe (P) als nicht valide, falls wenigstens eines der Nebenkriterien nicht erfüllt wird.

4. Computerimplementiertes Verfahren nach Anspruch 1,
ferner aufweisend
- Bestimmen des letzten Fluoreszenzwertes (LFL) der zu klassifizierenden Real-Time PCR-Amplifikationskurve (K1)
- sowie Klassifizieren der Real-Time PCR-Amplifikationskurve (K1) der zugehörigen Probe (P) als eine Positivkurve in Abhängigkeit des letzten Fluoreszenzwertes (LFL) der zu klassifizierenden Real-Time PCR-Amplifikationskurve.

5. Vorrichtung (V) zur qualitativen Auswertung von Real-Time PCR Daten,
aufweisend
- eine Speichereinheit (SP) zum Bereitstellen einer zu klassifizierenden Real-Time PCR-Amplifikationskurve (K1) einer zugehörigen Probe (P),
- sowie eine Recheneinheit (R) zum Bestimmen eines Gütemaßes (RG), welches eine Ähnlichkeit der zu klassifizierenden Real-Time PCR-Amplifikationskurve (K1) zu einer Geraden (GER) indiziert, als ein Gütemaß einer linearen Regression in Bezug auf die zu klassifizierenden Real-Time PCR-Amplifikationskurve (K1),
- ferner zum Bestimmen eines ersten Kriteriums durch Überprüfen, ob das Gütemaß (RG) der linearen Regression den ersten Vorgabewert (VW1) unterschreitet,
- sowie ferner zum Bestimmen einer Wertefolge (MK1`), welche eine Steigung der zu klassifizierenden Real-Time PCR-Amplifikationskurve (K1) indiziert, sowie zum Bestimmen eines zweiten Kriteriums, ob die Wertefolge (MK1') einen zweiten Vorgabewert (VW2) übersteigt,
wobei die Recheneinheit (R) eine Klassifikation der Real-Time PCR-Amplifikationskurve (K1) der zugehörigen Probe (P) als eine Negativkurve oder als eine Positivkurve vornimmt
und wobei die Recheneinheit (R) die Real-Time PCR-Amplifikationskurve (K1) der zugehörigen Probe (P) in dem Fall als eine Positivkurve klassifiziert, dass alle Kriterien erfüllt sind.

6. Computerprogrammprodukt, umfassend Befehle, die bei der Ausführung des Programms durch einen Computer diesen veranlassen, das Verfahren nach Anspruch 1 auszuführen.

## Claims

1. A computer-implemented method for the qualitative evaluation of real-time PCR data (DT),
wherein a real-time PCR amplification curve (K1) of an associated sample (P) is classified as a negative curve or as a positive curve, comprising
- providing the real-time PCR amplification curve (K1) to be classified of the associated sample (P),
- determining a quality metric (RG), which indicates a similarity of the real-time PCR amplification curve (K1) to be classified to a straight line (GER), as a quality metric of a linear regression with respect to the real-time PCR amplification curve (K1) to be classified,
- determining a first criterion by checking whether the quality metric (RG) of the linear regression falls below the first predefined value (VW1),
- determining a value sequence (MK1'), which indicates a slope of the real-time PCR amplification curve (K1) to be classified, and determining a second criterion as to whether the value sequence (MK1') exceeds a second predefined value (VW2),
- classifying the real-time PCR amplification curve (K1) of the associated sample (P) as a positive curve in the case that all criteria are met.

2. The computer-implemented method as claimed in claim 1,
furthermore comprising determining a criterion as to whether the value sequence (MK1'), which indicates a slope of the real-time PCR amplification curve to be classified, exceeds a third predefined value (VW3),
- classifying the real-time PCR amplification curve of the associated sample as a positive curve in the case that all criteria are met.

3. The computer-implemented method as claimed in claim 1,
furthermore comprising
- providing a real-time PCR amplification curve (P1) of a positive control sample (PK) and a real-time PCR amplification curve (N1) of a negative control sample (NK),
- determining a first secondary criterion as to whether the last fluorescence value of the real-time PCR amplification curve (P1) of the positive control sample (PK) has exceeded a minimum value (MIW),
- determining a second secondary criterion as to whether the last fluorescence value of the real-time PCR amplification curve (N1) of the negative control sample (NK) has not exceeded a maximum value (MAW),
- classifying the real-time PCR amplification curve (K1) of the associated sample (P) as not valid if at least one of the secondary criteria is not met.

4. The computer-implemented method as claimed in claim 1,
furthermore comprising determining the last fluorescence value (LFL) of the real-time PCR amplification curve (K1) to be classified,
- and classifying the real-time PCR amplification curve (K1) of the associated sample (P) as a positive curve as a function of the last fluorescence value (LFL) of the real-time PCR amplification curve to be classified.

5. A device (V) for the qualitative evaluation of real-time PCR data,
including
- a storage unit (SP) for providing a real-time PCR amplification curve (K1) to be classified of an associated sample (P),
- and a processing unit (R) for determining a quality metric (RG), which indicates a similarity of the real-time PCR amplification curve (K1) to be classified to a straight line (GER), as a quality metric of a linear regression with respect to the real-time PCR amplification curve (K1) to be classified,
- furthermore for determining a first criterion by checking whether the quality metric (RG) of the linear regression falls below the first predefined value (VW1),
- and furthermore for determining a value sequence (MK1'), which indicates a slope of the real-time PCR amplification curve (K1) to be classified, and for determining a second criterion as to whether the value sequence (MK1') exceeds a second predefined value (VW2),
wherein the processing unit (R) classifies the real-time PCR amplification curve (K1) of the associated sample (P) as a negative curve or as a positive curve,
and wherein the processing unit (R) classifies the real-time PCR amplification curve (K1) of the associated sample (P) as a positive curve in the case that all criteria are met.

6. A computer program product, comprising commands which, upon the execution of the program by a computer, cause it to carry out the method as claimed in claim 1.

## Revendications

1. Procédé informatisé d'évaluation qualitative de données PCR (DT) en temps réel, une classification d'une courbe d'amplification PCR en temps réel (K1) d'un échantillon associé (P) étant effectuée sous la forme d'une courbe négative ou d'une courbe positive, ledit procédé comportant les étapes suivantes :
- fournir la courbe d'amplification PCR en temps réel (K1) à classer de l'échantillon associé (P),
- déterminer une mesure de qualité (RG) qui indique une similarité entre la courbe d'amplification PCR en temps réel (K1) à classer et une droite (GER), comme mesure de qualité d'une régression linéaire par rapport à la courbe d'amplification PCR en temps réel (K1) à classer,
- déterminer un premier critère par vérification que la mesure de qualité (RG) de la régression linéaire devient inférieure à la première valeur par défaut (VW1),
- déterminer une séquence de valeurs (MK1'), qui indique une pente de la courbe d'amplification PCR en temps réel (K1) à classer, et déterminer un deuxième critère permettant de savoir si la séquence de valeurs (MK1') devient supérieure à une deuxième valeur par défaut (VW2),
- classer la courbe d'amplification PCR en temps réel (K1) de l'échantillon associé (P) comme courbe positive dans le cas où tous les critères sont remplis.

2. Procédé informatisé selon la revendication 1, comportant en outre les étapes suivantes :
- déterminer un critère permettant de savoir si la séquence de valeurs (MK1'), qui indique une pente de la courbe d'amplification PCR en temps réel à classer, devient supérieure à une troisième valeur par défaut (VW3),
- classer la courbe d'amplification PCR en temps réel de l'échantillon associé comme courbe positive dans le cas où tous les critères sont remplis.

3. Procédé informatisé selon la revendication 1, comportant en outre les étapes suivantes
- fourniture une courbe d'amplification PCR en temps réel (P1) d'un échantillon témoin positif (PK) et une courbe d'amplification PCR en temps réel (N1) d'un échantillon témoin négatif (NK),
- déterminer un premier critère secondaire permettant de savoir si la dernière valeur de fluorescence de la courbe d'amplification PCR en temps réel (P1) de l'échantillon témoin positif (PK) est devenue supérieure à une valeur minimale (MIW),
- déterminer un deuxième critère secondaire permettant de savoir si la dernière valeur de fluorescence de la courbe d'amplification PCR en temps réel (N1) de l'échantillon témoin négatif (NK) n'est pas devenue supérieure à une valeur maximale (MAW),
- classer la courbe d'amplification PCR en temps réel (K1) de l'échantillon associé (P) comme non valide si au moins un des critères secondaires n'est pas rempli.

4. Procédé informatisé selon la revendication 1, comportant en outre les étapes suivantes :
- déterminer la dernière valeur de fluorescence (LFL) de la courbe d'amplification PCR en temps réel (K1) à classer
- et classer la courbe d'amplification PCR en temps réel (K1) de l'échantillon associé (P) comme courbe positive en fonction de la dernière valeur de fluorescence (LFL) de la courbe d'amplification PCR en temps réel à classer.

5. Dispositif (V) d'évaluation qualitative de données PCR en temps réel, ledit dispositif comportant
- une unité de stockage (SP) destinée à fournir une courbe d'amplification PCR en temps réel (K1) à classer d'un échantillon associé (P),
- et une unité de calcul (R) destinée à déterminer une mesure de qualité (RG) qui indique une similarité entre la courbe d'amplification PCR en temps réel (K1) à classer et une droite (GER), comme mesure de qualité d'une régression linéaire par rapport à une courbe d'amplification PCR en temps réel (K1) à classer,
- également à déterminer un premier critère par vérification que la mesure de qualité (RG) de la régression linéaire devient inférieure à la première valeur par défaut (VW1),
- et également à déterminer une séquence de valeurs (MK1') qui indique une pente de la courbe d'amplification PCR en temps réel (K1) à classer, et à déterminer un deuxième critère permettant de savoir si la séquence de valeurs (MK1') devient supérieure à une deuxième valeur par défaut (VW2),
l'unité de calcul (R) effectuant une classification de la courbe d'amplification PCR en temps réel (K1) de l'échantillon associé (P) comme courbe négative ou comme courbe positive
et l'unité de calcul (R) classant la courbe d'amplification PCR en temps réel (K1) de l'échantillon associé (P) comme courbe positive dans le cas où tous les critères sont remplis.

6. Progiciel comprenant des instructions qui, lorsque le programme est exécuté par un ordinateur, ordonnent à celui-ci de mettre en oeuvre le procédé selon la revendication 1.
